# EUROPEAN PATENT APPLICATION

(11) **EP 4 473 910 A1**
(43) Date of publication of application: **11.12.2024**
(21) Application number: 23177752.5
(22) Date of filing: 06.06.2023
(51) Int. Cl.: A61B 5/287, A61B 5/316, A61B 5/318, A61B 5/349, A61B 5/00

(54) **METHOD FOR ANALYZING AN ELECTROGRAM**

(71) Applicant: CathVision ApS, 2200 Copenhagen (DK)
(72) Inventor: PAAMAND, Rune, 40468 Düsseldorf (DE)
(74) Representative: Gottschald Patentanwälte Partnerschaft mbB

(57) **Abstract**

The invention relates to a method for analyzing an electrogram (1), via a control system, wherein the electrogram (1) has been recorded via a catheter inserted into a human body, wherein the catheter comprises multiple electrodes, wherein at least one channel (K) of the electrogram (1) has been recorded by, preferably between, the electrodes, wherein the control system detects activation candidates (3) in a first channel (K_a), wherein multiple different potential sequences (4) of activation candidates (3) can be defined along the time dimension (5) of the first channel (K_a), wherein the control system assigns costs comprising one or more independent cost components to the potential sequences (4), wherein the control system selects a sequence (2) of dominant activations from the potential sequences (4) of the first channel (K_a), wherein the control system selects the sequence (2) of dominant activations that fulfills an optimization criterion based on the costs of the potential sequences (4). It is proposed that the control system assigns cost components to activation candidates (3), that the costs of the potential sequences (4) comprise the cost components of the activation candidates (3) defining the respective potential sequence (4).

## Description

The present invention relates to a method for analyzing an electrogram according to the general part of claim 1 and to a control system configured to perform the proposed method according to claim 14.

The present method is particularly concerned with atrial fibrillation and atrial flutter. Electrically, atrial fibrillation is chaotic activation of muscle cells of the atria. During atrial fibrillation, the atria only minimally contribute to the function of the heart. Atrial fibrillation, therefore, reduces the output of the heart but is not imminently dangerous. However, when becoming chronic, atrial fibrillation is correlated with increased morbidity and mortality.

During atrial fibrillation competing activation rhythms can exist in the atria. Some of these rhythms may be stable while others emerge and disappear quickly. Different methods for assessing the severity of atrial fibrillation and evaluating treatment options include the detection of rhythms in one or both atria by detecting sequences representative of the rhythm. However, the more chaotic and complex the atrial fibrillation is, the more competing rhythms may exist at a given region in an atrium. The measurements of an electrode placed in the atrium will therefore include different rhythms of local cell depolarizations and electrical current conducted through electrical conduction not based on cell depolarization. Detecting local activations and deriving one or more dominant local rhythms allows for a better understanding of the atrial fibrillation, for example during 3D mapping procedures. However, detecting which activation is local and which rhythm or rhythms lead to those activations is challenging especially during atrial fibrillation.

The known prior art (WO 2015/149153 A1) that builds the basis of the invention is related to a method according to the general part of claim 1.

WO 2015/149153 A1 proposes a method of annotation where in a first computation step activation candidates are identified and an overall estimate of a mean activation interval ("jump length" below) is calculated. Then in a second computation step a sequence of the activation candidates is identified such that they minimize the overall difference between individual activation intervals and the estimate of the overall activation interval. The prior art achieves this by using a cost function which assigns costs to jumps between activation candidates. In particular, costs are assigned to every possible jump between activation candidates and the path through time with the lowest cost is identified. This overall minimization may be achieved by computational methods for shortest path search known from computational graph theory. One example of a suitable shortest path search is the Djikstra algorithm.

The known algorithm provides a good basis for the annotation of a dominant rhythm in a channel of a, here intracardiac, electrogram. Nevertheless, there is still room for improvement of the algorithm to improve the detection of local rhythms.

The invention is based on the problem of improving the known method such that a further optimization regarding the named challenge is reached.

The above-noted object is solved by the features of the characterizing part of claim 1.

The main realization of the present invention is that further information can be used to decide which activation candidates form a physiologically valid sequence. This further information comes in the form of characteristics of the activation candidates themselves. It is proposed to translate these characteristics into costs of activation candidates. Exemplarily using a cost function to find the sought sequence, the path through time then depends on the cost of each activation candidate being part of the sequence, instead of or in addition to costs for the jumps between the activation candidates.

Several characteristics usable for such costs have been identified. For example, the morphology of activation candidates of a rhythm may have a higher similarity than the morphology of activation candidates of different rhythms. Further, crosschannel similarities between activation candidates, in particular their timing, may be used. Local activations travel slower through channels than far field potentials, which often appear at essentially the same time in some or all channels. Competing with this, local activation sequences that do not appear in more than one channel or in only a few channels may be regionally confined activation sequences while local activation sequences that can be found on more channels or channels spaced further apart may be the sought dominant activation rhythms. The question which rhythm is sought may also depend on the clinical use, which can be coded into the costs, too.

It is therefore proposed to assign cost components to activation candidates to identify a sequence based on characteristics of the activation candidates.

Here and preferably, the electrogram is intracardiac electrogram. The term "intracardiac" is to be understood broadly and relates to measurements inside the human heart and in direct vicinity to it, for example in the pulmonary veins, from the inside. Alternatively, the electrogram may have been recorded by any other invasive method. In particular, epicardial measurements are comprised.

In detail, it is proposed that the control system assigns cost components to activation candidates, that the costs of the potential sequences comprise the cost components of the activation candidates defining the respective potential sequence.

In a preferred embodiment according to claim 2, cost components are also assigned to jumps between activation candidates. This allows optimizing for a consistent jump length if looking for rhythmic sequences and at the same time taking into account the characteristics of the activation candidates.

In a preferred embodiment according to claim 3, some cost components depend on data from a second channel. In particular, cost components assigned to activation candidates may depend on a distance in time between these activation candidates and activation candidates from another channel. Defining costs in this manner optimizes consistency between electrodes. Such a consistency is particularly advantageous in situations where competing activation rhythms exist.

According to claim 4, the cost components depending on the second channel may comprise time-dependent and/or time-independent cost components. Time dependent cost components are cost components that depend on the timing of the activation candidate such that hypothetically changing the time stamp of the activation candidate with all other things equal would have an influence on the cost component. Time-independent cost components would not change if only the time stamp of an activation candidate was changed.

If no mapping system is used, the control system may not have any data about relative positions of the electrodes and respectively channels. It is still possible to derive which electrodes are physically near to each other by using the ordering of the electrodes and channels (claim 5). It is for example rather unlikely that a rhythm which can be traced through channels I to III but not channels IV and V appears again in channel VI.

Claim 6 relates to a preferred embodiment in which unipolar signals and bipolar signals are combined to detect rhythms by using cost components depending on unipolar channels as cost components of activation candidates in a bipolar channel. This idea is based on the fact that cell depolarization is always negative and therefore the down deflection of the unipolar electrode can be included in the cost components advantageously to detect local activations.

To detect far field potentials and reduce their influence on the result of the analysis, cost components may depend on data from a coronary sinus electrode and/or a surface ECG electrode (claim 7). The coronary sinus electrode may be used to detect actual beats of the heart and increase the cost components of activation candidates soon after the heartbeats which may have a higher chance of being far field interference.

Embodiments according to claim 8 relate to basing cost components for the activation candidates on a morphology of the activation candidates. According to claim 9, a, in particular morphological, similarity between activation candidates is included in the cost components, for example by basing cost components of jumps between activation candidates on the morphological similarity of the two activation candidates that the jump connects.

Claim 10 relates to the possibility of static and/or dynamic cost components. Static cost components are independent of the sequence while dynamic cost components change with the sequence. For example, the cost components of a jump may depend on the mean jump length of other jumps in the sequence such that the overall similarity of jump lengths in a sequence is optimized rather than the similarity of jumps lengths to a predefined mean jump length.

According to claims 11 and 12 the channels may be analyzed iteratively and the processing of a channel may depend on a previously processed channel. Possibly, more than one iteration can be performed for at least one channel.

In an embodiment according to claim 13, the analysis is performed cyclically, in particular in real time, and the control system analyzes, for example each second, the channels cyclically. As dominant rhythms can change, it is preferably possible that detected sequences change for the past with a new analysis cycle. The result of a previous analysis may be included in the costs to lower the probability of detecting two different rhythms and frequently changing which is detected. Including past analysis in the costs makes an artificial hurdle for changes between detected rhythms.

Another teaching according to claim 14, which is of equal importance, relates to a control system configured to perform the proposed method, wherein the control system is configured to receive and/or measure the electrogram.

All explanations given with regard to the proposed method are fully applicable.

In the following, embodiments of the invention are explained with respect to the drawing. The drawing shows in
- Fig. 1,: finding a sequence through analysis of the cost components,
- Fig. 2,: iteratively deriving cost components for multiple channels according to one embodiment and
- Fig. 3,: deriving cost components depending on another channel in a time-dependent manner.

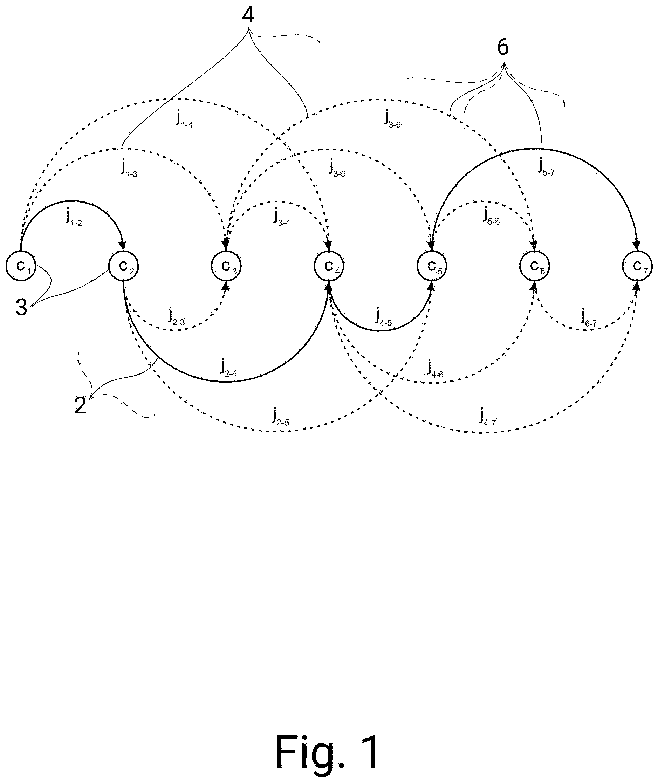

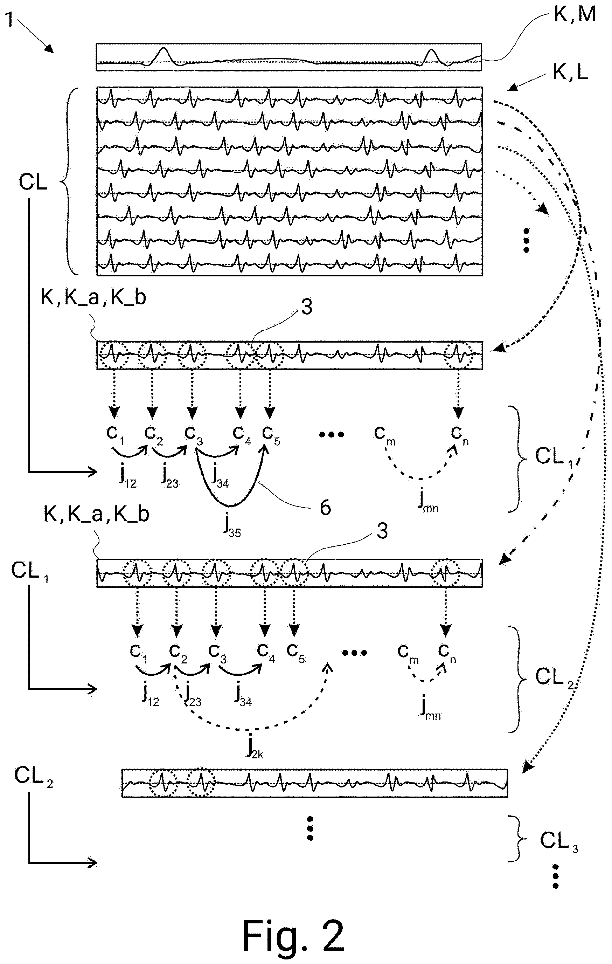

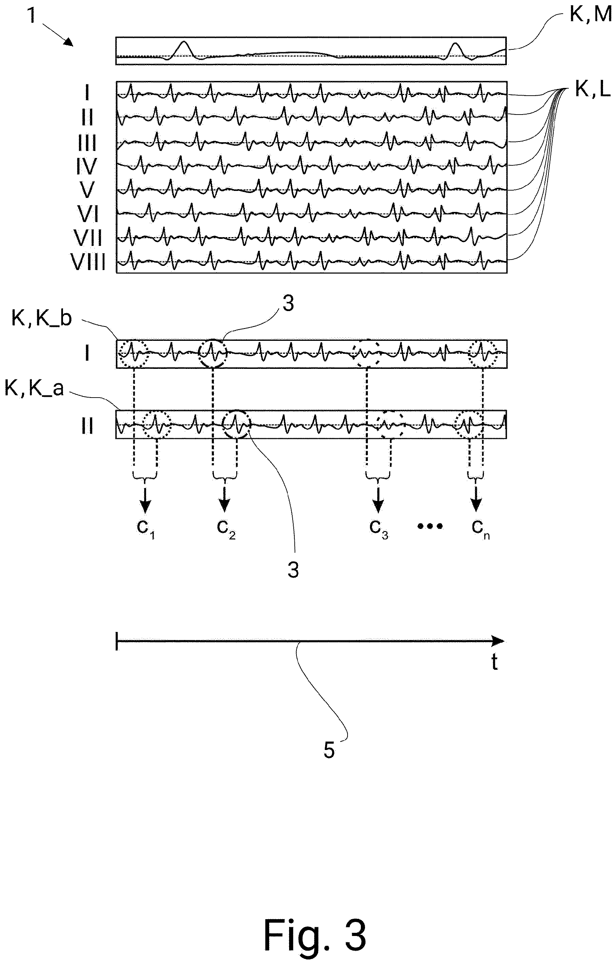

The figures show an embodiment of the proposed method for analyzing a, here and preferably intracardiac, electrogram 1. Fig. 1 shows generally how a cost function is used to determine a sequence 2 from a number of nodes and edges between nodes. Figs. 2 and 3 show an exemplary section of an intracardiac electrogram 1 in a rather schematic manner. The shown intracardiac electrogram 1 comprises a coronary sinus channel M, depicted only very schematically, at the top and, purely exemplarily, 8 bipolar atrial channels L. A corresponding intracardiac electrogram 1 may have been recorded during a mapping procedure. Preferably, the intracardiac electrogram 1 has been recorded during atrial fibrillation.

The analysis is performed by a control system, for example a general purpose computer, a mapping system or a dedicated hardware device comprising a processor. The proposed method is therefore a computer-implemented method. Any method step described herein can therefore be performed by the control system and the control system may be adapted accordingly.

The electrogram 1 has been recorded via a catheter inserted into a human body. The catheter comprises multiple electrodes. At least one channel K of the electrogram 1 has been recorded by, preferably between, the electrodes. The channel K or channels K may be unipolar, recorded by one electrode of the catheter, or bipolar, recorded between two electrodes of the catheter.

Generally, the proposed method relates to detecting rhythms in form of sequences 2 in the electrogram 1, in particular in near real time during surgery. The control system may display the result of the analysis to a user, in particular a surgeon. The information can be used by the user for diagnostic purposes and/or for devising a treatment plan.

An electrode placed in an atrium will always measure local electrical depolarizations of cells and electrical potentials reaching the electrode through electrical conduction, also known as far field potentials or far field interference. During normal cardiac activity in a healthy atrium, the local activations comprise one rhythm corresponding to the beats of the atrium. During atrial fibrillation, different locations may have different rhythms and even one location may have competing rhythms. Further, random activations and activations from neighboring tissue will be visible in the channel K of a given electrode. To better understand the complex compound picture of the atrial fibrillation, it is desired to annotate a single or a small number of rhythms for a given channel K, for example for subsequently creating a map of the electrical activations of the atrium.

As part of the proposed method, the control system detects activation candidates 3 in a first channel K_a. The terms "first" and "second" are not limiting and only used to aid the understanding of the explanations. Any channel K may be the first channel K_a and any other channel K may be the yet to be explained second channel K_b. Activation candidates 3 are sections of the signal of the respective channel K comprising peaks with a certain characteristic or otherwise selected as possibly representing local depolarizations. The intracardiac electrogram 1 in Figs. 2 and 3 show a number of such activation candidates 3.

Multiple different potential sequences 4 of activation candidates 3 can be defined along the time dimension 5 of the first channel K_a. In principle, any combination of at least three, preferably at least 5, more preferably at least 10, activation candidates 3 being a subgroup of all activation candidates 3 may form a potential sequence 4. It should be understood that the number of potential sequences 4 is greater than 1 and that the sequence 2, being a subgroup of the activation candidates 3, does not comprise all activation candidates 3 of a channel K.

The control system assigns costs comprising one or more independent cost components to the potential sequences 4. Generally speaking, the costs of a potential sequence 4 are composed by the cost components of the potential sequence 4, in particular the cost components of the elements of the potential sequence 4. For example, a sequence 2 comprising several activation candidates 3 distributed over the time dimension 5 may comprise cost components assigned to the jumps 6 between the activation candidate 3, each jump 6 being the time distance between the activation candidate 3 and the next activation candidate 3 chosen of the potential sequence 4. The cost components assigned to the jumps 6 could simply be the length of the jump 6. Further exemplarily, the costs may be the sum of the cost components. It should be noted though that the length of the jumps 6 alone would not be a useful cost function as the sum of costs would then just be the time difference between the first and the last activation candidate 3.

The control system selects a sequence 2 of dominant activations from the potential sequences 4 of the first channel K_a, for example by selecting the potential sequence 4 with the lowest costs. More generally, the control system selects the sequence 2 of dominant activations that fulfills an optimization criterion based on the costs of the potential sequences 4. In particular, mathematically equivalent or similar optimization criterions to finding the minimum costs are possible.

Here and preferably, the first channel K_a is a bipolar channel K and/or an atrial channel L and/or not a coronary sinus channel M.

Proposed is that the control system assigns cost components to activation candidates 3. The costs of the potential sequences 4 then comprise the cost components of the activation candidates 3 defining the respective potential sequence 4. Fig. 1 shows a general example of how a sequence 2 may be selected. Fig. 1 shows 7 activation candidates 3 shown as nodes and some of the possible jumps 6 between the nodes. For a better understanding of the figure not all possible jumps 6 are shown. It may be the case that jumps 6 over a predefined length and/or jumps 6 below a predefined length are sorted out to reduce the number of potential sequences 4. Each of the activation candidates 3 has an assigned cost component "c", the index being the number of the activation candidate 3. As an optional addition, Fig. 1 also shows cost components assigned to the jumps 6 between nodes and marked with a "j" with an index indicating from which node to which node the jump 6 leads. The selected sequence 2 shown with a solid line for example would have costs of the sum of the cost components of the activation candidates 3 with the numbers 1, 2, 4, 5 and 7 and the cost components of the jumps 6 between nodes 1 and 2, 2 and 4, 4 and 5, 5 and 7.

While it is possible to use any mathematical equivalent of assigning cost components to nodes and still assign cost components to activation candidates 3, for example by making the cost components of jumps 6 dependent on their start and target nodes, thereby hiding the cost components of the nodes in the cost components of the jumps 6, it should still be understood that the cost components of activation candidates 3 depend on the respective activation candidate 3, irrespective of their mathematical representation and can be distinguished from cost components that depend on jumps 6, in particular jump lengths CL, which are independent of the activation candidates 3 themselves. Here and preferably, the cost components of the activation candidates 3 are independent of the timing of the activation candidate 3 within the channel K and/or independent of the position of the activation candidate 3 within a sequence 2.

Preferably, within a channel K, the cost components of the activation candidates 3 are time-invariant. Therefore, if for example three activation candidates 3 with a different morphology were placed at 0 ms, 100 ms, and 200 ms of a hypothetical channel K, if all cost components are time-invariant, the costs of the sequence 2 are the same if, hypothetically, the ordering of the activation candidates 3 was changed by swapping the locations of two of the activation candidates 3, all other things equal. Naturally, such a swap would be difficult to achieve in a real signal.

It should also be noted that costs including the cost components mentioned herein are not zero and not infinite or prohibitively high and therefore practically infinite. Such infinite or zero costs may exist in addition to the mentioned costs and in different repetitions of the method different fractions of the costs may be zero or infinite, but the costs mentioned herein and relevant for the present description are always costs that can, by design, realistically lead to choosing of the respective element of the sequence 2.

Fig. 2 and 3 show a preferred embodiment of an algorithm for selecting a sequence 2 from a number of activation candidates 3. In the following, this embodiment will be used as an example to explain further advantageous embodiments of the invention. In Fig. 2, as a first step, a first channel K_a, here the uppermost atrial channel L, is selected and processed first (uppermost arrow on the right). The control system detects activation candidates 3 (marked with circles) in the first channel K_a and optionally assigns a position in time to each of them based for example on the position of a main peak of the activation candidate 3. The detection of the activation candidates 3 may be done with known algorithms, for example based on peak detection, morphological detection or other methods. Going further downwards in Fig. 2, the control system assigns cost components c to the activation candidates 3.

According to one embodiment it is proposed that the control system assigns cost components to jumps 6 between activation candidates 3. This embodiment can also be seen in Fig. 2 with the cost components j. The costs of the potential sequences 4 then comprise the cost components of the jumps 6 between activation candidates 3 defining the respective potential sequence 4. The cost components assigned to jumps 6 may depend on a difference between lengths of the jumps 6 and an estimate of a mean jump length CL (also called cycle length). The mean jump length CL may be an expected jump length CL for an expected dominant rhythm. Known ways of estimating a mean jump length CL include the calculation of a dominant frequency and other methods using for example Fourier transformations. Fig. 2 shows that the mean jump length CL for the first channel K_a may be estimated from all atrial channels L. It is generally the case that the mean jump length CL for a channel K may be estimated from the same channel K or from multiple channels K or from another channel K as will be explained for the second channel K_b.

In one embodiment the potential sequences 4 are limited by defining a starting and/or ending zone for the sequences 2 and only considering sequences 2 with a first and/or last activation candidate 3 inside the respective zone.

With the given information it is already possible to select a sequence 2 from the potential sequences 4. It is also possible to process all channels K in this manner and therefore analyze the electrogram 1.

However, according to one embodiment it is proposed that the costs of the potential sequences 4 of the first channel K_a, in particular the cost components assigned to activation candidates 3, comprise cost components dependent on data derived from a second channel K_b. In this embodiment and at odds with the example given above, the channel K first analyzed in Fig. 2 is the second channel K_b and any of the other channels K is the first channel K_a. Alternatively, as will be explained, the uppermost channel K may be analyzed twice, in which case it is not relevant which channel K is defined as the first channel K_a. Fig. 2 shows that after analyzing the uppermost channel K (here the second channel K_b), a mean jump length CL estimate may be derived from the selected sequence 2 of the second channel K_b. This estimate is then used to find the sequence 2 in the first channel K_a by using data, here the mean jump length CL estimate, from another channel K.

Fig. 3 shows another preferred embodiment in which the first channel K_a, here the channel K with the number II, is analyzed by assigning cost components dependent on the second channel K_b (prior analyzed channel K with the number I) to the activation candidates 3 of the first channel K_a. Here and preferably, the cost components assigned to activation candidates 3 of the first channel K_a depend on activation candidates 3 of a selected sequence 2 of a previously analyzed second channel K_b. Fig. 3 shows how the cost components of activation candidates 3 may depend on a distance in time to activation candidates 3 of a second channel K_b. The chance that a sequence 2 actually represents the dominant rhythm is higher if a similar sequence 2 is found in other channels K.

Here and preferably, the first channel K_a and the second channel K_b are channels K from electrodes of the same catheter and/or bipolar channels K and/or atrial channels L and/or neither the first channel K_a nor the second channel K_b are a coronary sinus channel M.

According to one embodiment it is proposed, that the cost components dependent on data derived from a second channel K_b comprise time-independent cost components and/or time-dependent cost components. Time-independent cost components are the same independent of the timing of the respective element of the potential sequences 4 to which the cost component is assigned. Above example with the mean jump length CL is time-independent.

Generally said, preferably, the time-independent cost components depend on a difference between jump lengths CL of the first channel K_a and an estimate of a mean jump length CL of the second channel K_b.

Above example with the cost components of activation candidates 3 is time-dependent as the cost components depend on the timing of the activation candidate 3 with regard to activation candidates 3 of other channels K. Generally said, preferably, the time-dependent cost components depend on the timing of activation candidates 3 of the first channel K_a compared to timing of activation candidates 3 of the second channel K_b.

It is preferably the case that the second channel K_b is a neighboring channel K of the first channel K_a. The control system may use an ordering of the electrodes to derive which channels K are neighboring channels K. Usually, channel K or electrode numbers are chosen based on the physical position of the electrodes on the catheter. An easy way of estimating physical neighborhood of electrodes is therefore using the provided channel K order.

Another way of assigning cost components to potential sequences 4 is based on the unipolar signals of bipolar channels K. It may be the case that the first channel K_a is a bipolar channel K, that the costs of the potential sequences 4 of the first channel K_a, in particular the cost components assigned to activation candidates 3, comprise cost components dependent on data derived from one or both unipolar channels K of the electrodes of the bipolar channel K, in particular on data about a polarization of the electrode or electrodes. This embodiment is based on the realization that cell depolarization is always negative and the down deflection of an electrode is therefore indicative of whether an activation is local or not.

According to another embodiment it is proposed, that the costs of the potential sequences 4 of the first channel K_a, in particular the cost components assigned to activation candidates 3, comprise cost components dependent on data derived from a coronary sinus channel M and/or a surface ECG electrode. A coronary sinus electrode may be used to estimate which activation candidates 3 are more likely to be far field interference. The cost components dependent on data derived from a coronary sinus channel M and/or the surface ECG electrode may therefore depend on data about the time distance between activation candidates 3 in the first channel K_a and ventricular beats detected in the coronary sinus channel M and/or the surface ECG electrode. Alternatively to increasing their cost components, activation candidates 3 that are highly likely to be far field interference can be removed completely such that they cannot be chosen as part of the sequence 2.

It may also be the case that the cost components assigned to activation candidates 3 comprise cost components dependent on a morphology of the respective activation candidate 3. The morphology may include descriptive features such as amplitude and/or dV/dt slopes and/or RMS values and/or frequency content and/or correlation coefficients, etc. or the cost may be derived from the output of an algorithm, such as a machine learning model, taking the electrogram interval as at least one of its inputs.

In another embodiment it may be the case that a clinical use of the analysis is coded into the cost components, in particular the cost components assigned to activation candidates 3. The clinical use may be input, in particular chosen from a list, by a user.

Further, the costs of the potential sequences 4 may comprise cost components dependent on a measure of, in particular morphological, similarity between activation candidates 3 of the first channel K_a and/or of a selected sequence 2 of a previously analyzed second channel K_b. Similar activation candidates 3 may be more likely to form a valid sequence 2. Using the activation candidates 3 of a sequence 2 already defined as valid reduces the number of bad morphologies in the comparison.

Another possibility is that the costs of the potential sequences 4 of the first channel K_a, in particular the cost components assigned to activation candidates 3 and/or jumps 6, comprise static cost components independent of the potential sequence 4 and/or dynamic cost components dependent on the potential sequence 4. It is computationally easier in many cases not to use cost components dependent on the sequence 2 itself, but, in particular for morphological comparisons, comparing the morphology of activation candidates 3 of a sequence 2 and then adapting the cost of that sequence 2 may be advantageous.

Preferably, the dynamic cost components assigned to jumps 6 depend on a measure of similarity of jump lengths CL of the respective potential sequence 4, thereby rewarding jumps 6 of similar length. Additionally or alternatively, the dynamic cost components assigned to activation candidates 3 may depend on a measure of similarity of activation candidates 3 of the respective potential sequence 4.

It can be summarized that here and preferably the analysis of the channels K is not a strict forward-search but rather uses a global optimization. Here and preferably, a graph search algorithm, for example Dijkstra, is used to select the sequence fulfilling the optimization criterion. Generally, preferably a global optimization algorithm is used on the costs of a given channel to select the sequence fulfilling the optimization criterion.

For computational efficiency it may nevertheless be the case that the control system analyzes channels K iteratively and that the analysis of channels K depends on the analysis of previously analyzed channels K. Preferably, the cost components of the potential sequences 4 of a channel K depend on the analysis of a previously analyzed channel K. More preferably, at least one channel K is analyzed twice at least sometimes, in particular if a measure of precision of the analysis has not been met, whereby the second analysis depends on the analysis of channels K analyzed after the first analysis. This may particularly be the case for the channel K analyzed first.

As mentioned, according to one embodiment it is proposed that the time-dependent cost components depend on the timing of activation candidates 3 of the first channel K_a compared to timing of activation candidates 3 of a selected sequence 2 of a previously analyzed second channel K_b.

Turning towards the clinical use, it may be the case that the control system cyclically receives updated channels K and analyzes the updated channels K, that updated channels K comprise a section previously analyzed and a new section and that the selection of activation candidates 3 in the previously analyzed section sometimes changes with the analysis of the updated channels K. This embodiment is thought to be very unusual. Normally, an analysis would not change annotations made in a previous round of analysis. However, the dominating rhythm in a given region may change over time and this change may have announced itself in the previous data while not yet making it into the selected sequence 2. Retrospectively relabeling the data allows a quicker reaction to and a better detection of such changes.

Preferably, the cost components of the potential sequences 4 in the analysis of the updated channels K comprise cost components dependent on the selection of activation candidates 3 in the previously analyzed section. By providing a kind of memory of previous labels in the cost components, frequent changes back and forth between two similarly dominant rhythms are made less likely.

Another teaching which is of equal importance relates to a control system configured to perform the method according to one of the preceding claims, wherein the control system is configured to receive and/or measure the electrogram 1. The control system may comprise a processor and/or a memory. The control system may be a local unit with a processor, possibly a user interface and the like. It may also comprise in one embodiment a cloud processor. It is therefore not necessary for the control system to be confined to a single device. The control system may comprise an interface for the catheter. The control system is configured to perform the proposed method.

## Claims

1. Method for analyzing an electrogram (1), via a control system, wherein the electrogram (1) has been recorded via a catheter inserted into a human body, wherein the catheter comprises multiple electrodes, wherein at least one channel (K) of the electrogram (1) has been recorded by, preferably between, the electrodes,
wherein the control system detects activation candidates (3) in a first channel (K_a),
wherein multiple different potential sequences (4) of activation candidates (3) can be defined along the time dimension (5) of the first channel (K_a), wherein the control system assigns costs comprising one or more independent cost components to the potential sequences (4),
wherein the control system selects a sequence (2) of dominant activations from the potential sequences (4) of the first channel (K_a), wherein the control system selects the sequence (2) of dominant activations that fulfills an optimization criterion based on the costs of the potential sequences (4),
**characterized in**
**that** the control system assigns cost components to activation candidates (3), that the costs of the potential sequences (4) comprise the cost components of the activation candidates (3) defining the respective potential sequence (4).

2. Method according to claim 1, **characterized in that** the control system assigns cost components to jumps (6) between activation candidates (3), that the costs of the potential sequences (4) comprise the cost components of the jumps (6) between activation candidates (3) defining the respective potential sequence (4), preferably, that the cost components assigned to jumps (6) depend on a difference between lengths of the jumps (6) and an estimate of a mean jump length (CL).

3. Method according to claim 1 or 2, **characterized in that** the costs of the potential sequences (4) of the first channel (K_a), in particular the cost components assigned to activation candidates (3), comprise cost components dependent on data derived from a second channel (K_b), preferably, that the first channel (K_a) and the second channel (K_b) are channels (K) from electrodes of the same catheter and/or bipolar channels (K) and/or atrial channels (L) and/or neither the first channel (K_a) nor the second channel (K_b) are a coronary sinus channel (M).

4. Method according to claim 3, **characterized in that** the cost components dependent on data derived from a second channel (K_b) comprise time-independent cost components and/or time-dependent cost components,
preferably, that the time-independent cost components depend on a difference between jump lengths (CL) of the first channel (K_a) and an estimate of a mean jump length (CL) of the second channel (K_b), and/or,
that the time-dependent cost components depend on the timing of activation candidates (3) of the first channel (K_a) compared to timing of activation candidates (3) of the second channel (K_b).

5. Method according to claim 3 or 4, **characterized in that** the second channel (K_b) is a neighboring channel (K) of the first channel (K_a), preferably, that the control system uses an ordering of the electrodes to derive which channels (K) are neighboring channels (K).

6. Method according to one of the preceding claims, **characterized in that** the first channel (K_a) is a bipolar channel (K), that the costs of the potential sequences (4) of the first channel (K_a), in particular the cost components assigned to activation candidates (3), comprise cost components dependent on data derived from one or both unipolar channels (K) of the electrodes of the bipolar channel (K), in particular on data about a polarization of the electrode or electrodes.

7. Method according to one of the preceding claims, **characterized in that** the costs of the potential sequences (4) of the first channel (K_a), in particular the cost components assigned to activation candidates (3), comprise cost components dependent on data derived from a coronary sinus channel (M) and/or a surface ECG electrode, in particular on data about the time distance between activation candidates (3) in the first channel (K_a) and ventricular beats detected in the coronary sinus channel (M) and/or the surface ECG electrode.

8. Method according to one of the preceding claims, **characterized in that** the cost components assigned to activation candidates (3) comprise cost components dependent on a morphology of the respective activation candidate (3).

9. Method according to one of the preceding claims, **characterized in that** the costs of the potential sequences (4) comprise cost components dependent on a measure of, in particular morphological, similarity between activation candidates (3) of the first channel (K_a).

10. Method according to one of the preceding claims, **characterized in that** the costs of the potential sequences (4) of the first channel (K_a), in particular the cost components assigned to activation candidates (3) and/or jumps (6), comprise static cost components independent of the potential sequence (4) and/or dynamic cost components dependent on the potential sequence (4), preferably, that the dynamic cost components assigned to jumps (6) depend on a measure of similarity of jump lengths (CL) of the respective potential sequence (4), and/or, that the dynamic cost components assigned to activation candidates (3) depend on a measure of similarity of activation candidates (3) of the respective potential sequence (4).

11. Method according to one of the preceding claims, **characterized in that** the control system analyzes channels (K) iteratively and the analysis of channels (K) depends on the analysis of previously analyzed channels (K), preferably, that the cost components of the potential sequences (4) of a channel (K) depend on the analysis of a previously analyzed channel (K), preferably, that at least one channel (K) is analyzed twice at least sometimes, in particular if a measure of precision of the analysis has not been met, whereby the second analysis depends on the analysis of channels (K) analyzed after the first analysis.

12. Method according to claims 4 and 11, **characterized in that** the time-dependent cost components depend on the timing of activation candidates (3) of the first channel (K_a) compared to timing of activation candidates (3) of a selected sequence (2) of a previously analyzed second channel (K_b).

13. Method according to one of the preceding claims, **characterized in that** the control system cyclically receives updated channels (K) and analyzes the updated channels (K), that updated channels (K) comprise a section previously analyzed and a new section, that the selection of activation candidates (3) in the previously analyzed section sometimes changes with the analysis of the updated channels (K), preferably, that the cost components of the potential sequences (4) in the analysis of the updated channels (K) comprise cost components dependent on the selection of activation candidates (3) in the previously analyzed section.

14. Control system configured to perform the method according to one of the preceding claims, wherein the control system is configured to receive and/or measure the electrogram (1).
